# EUROPÄISCHE PATENTSCHRIFT

(11) **EP 3 717 125 B1**
(45) Veröffentlichungstag und Bekanntmachung des Hinweises auf die Patenterteilung: **21.04.2021**
(21) Anmeldenummer: 19800917.7
(22) Anmeldetag: 15.10.2019
(51) Int. Cl.: C12M 3/06, B01L 3/00, B01L 3/12, C12M 1/00, C12M 1/24, B01L 3/10

(54) **BEGASUNGSVORRICHTUNG**
GASSING DEVICE
DISPOSITIF DE FUMIGATION

(30) Priorität: 06.12.2018 DE 102018131184
(43) Veröffentlichungstag der Anmeldung: 07.10.2020
(73) Patentinhaber: Adolf Kühner AG, 4127 Birsfelden (CH)
(72) Erfinder: ZUMBRUNNEN, Simon Paul, 3414 Oberburg (CH); BÜRGIN, Tim, 4410 Liestal (CH); ANDERLEI, Tibor, 79379 Müllheim (DE)
(74) Vertreter: Kohlmann, Kai
(86) Internationale Anmeldenummer: PCT/EP2019/077858
(87) Internationale Veröffentlichungsnummer: WO 2020/114655

(56) Entgegenhaltungen:
- EP-A2- 0 905 229
- US-A1- 2002 185 186

## Beschreibung

Die Erfindung betrifft eine Begasungsvorrichtung für einen Schüttelkolben.

Im Stand der Technik ist es bekannt, Kultivierungen mit Schüttelkolben in Schüttel-Inkubatoren unter vorgegebenen Bedingungen, wie einer bestimmten Temperatur und Feuchtigkeit, sowie einer definierten Gasatmosphäre durchzuführen. Die Adolf Kühner AG Dinkelbergstr. 1, 4127 BIRSFELDEN (Basel), Schweiz, stellt u.a. unter der Bezeichnung ISF1-X einen derartigen Schüttel-Inkubator her.

Eine Begasung mit verschiedenen Gasen oder Gasgemischen ist insbesondere bei der automatisierten Erfassung von Prozessparametern mikrobieller, biochemischer, enzymatischer und chemischer Reaktionen von Kulturen sowie pflanzlicher, tierischer und humaner Zellen in Schüttelkolben erforderlich, die bis zum Abschluss der Reaktion unterbrechungslos geschüttelt werden. Als Parameter der Kulturen werden beispielsweise die Sauerstoff-Transferrate (OTR) und die Kohlendioxid-Transferrate (CTR) erfasst und daraus die Parameter Respirationsquotient (RQ) und maximale spezifische Wachstumsrate (µₘₐₓ) abgeleitet.

Die Schüttelkolben weisen zur Begasung in dem Inkubator jeweils eine Verschlusskappe mit einer einen Mantel begrenzenden Decke auf, in der ein Gasdurchgang angeordnet ist. Unterhalb des Gasdurchgangs befindet sich an der Innenseite der Decke ein Sterilfilter. Durch den Durchgang hindurch wird die Kultur innerhalb des Schüttelkolbens begast. Die Begasung erfolgt üblicherweise mittels eines Gemisches aus Kohlendioxid und Luft. Die Kultivierung innerhalb des Inkubators erfordert die Aufrechterhaltung der Temperatur, Feuchtigkeit und Zusammensetzung der definierten Gasatmosphäre innerhalb des Inkubators. Wird während der Kultivierung der Inkubator geöffnet, kann sich die Temperatur, Feuchtigkeit und Zusammensetzung der Gasatmosphäre innerhalb des Inkubators und infolgedessen innerhalb der begasten Schüttelkolben ändern EP0905229A2 und US2002/185186 sind ebenfalls Stand der Technik.

Ausgehend von diesem Stand der Technik liegt der Erfindung die Aufgabe zugrunde, eine Begasungsvorrichtung für einen Schüttelkolben für den Betrieb in einem Inkubator zu schaffen, die eine Begasung der Kultur innerhalb des Schüttelkolbens unabhängig von der Zusammensetzung und Feuchtigkeit der Gasatmosphäre innerhalb des Inkubators ermöglicht.

Die Lösung dieser Aufgabe beruht auf dem Gedanken, jeden Schüttelkolben über eine lösbar mit der Verschlusskappe verbundene, einfach handhabbare Begasungsklammer individuell und unabhängig von der Feuchtigkeit und Zusammensetzung der Gasatmosphäre in dem Inkubator zu begasen. Im Einzelnen wird die Aufgabe durch eine Begasungsvorrichtung mit den Merkmalen des Anspruchs 1 gelöst. Vorteilhafte Ausgestaltungen der Erfindung ergeben sich aus den Unteransprüchen.

Die Entkopplung der Begasung in jedem Schüttelkolben erfolgt mittels einer lösbar mit der Verschlusskappe des Schüttelkolbens verbundenen Begasungsklammer mit mindestens zwei elastischen Klemmbacken und einem die Klemmbacken miteinander verbindenden Steg, wobei die Klemmbacken auf den Mantel der Verschlusskappe eine Klemmkraft ausüben. Die Klammer lässt sich unproblematisch auf die Verschlusskappe aufsetzen und wird durch die elastischen Klemmbacken sicher gehalten. Der Steg der Begasungsklammer verfügt über eine Ausnehmung an seiner Innenseite, wobei die Ausnehmung bei korrekt aufgesetzter Begasungsklammer oberhalb des Gasdurchgangs in der Verschlusskappe angeordnet ist. Zusammen mit dem Sterilfilter bildet die gegen die Verschlusskappe abgedichtete Ausnehmung einen Gasraum. Mittels eines Einlasses zur Zufuhr von Gas in die Ausnehmung und einem Auslass für das Austreten von Gas aus der Ausnehmung kann oberhalb des Sterilfilters in dem Gasraum eine individuelle Gasatmosphäre für die Begasung der Kultur in den jeweiligen Schüttelkolben erzeugt werden.

Über den Einlass zur Zufuhr von Gas wird der Schüttelkolben mit einer Gasversorgung, vorzugsweise einer Gasmischbatterie, verbunden. Die Gasmischbatterie ist mit mehreren Gasquellen für unterschiedliche Gase verbunden. Bei den Gasquellen handelt es sich beispielsweise um Druckgasflaschen für Luft, Sauerstoff, Kohlendioxid und Stickstoff. Um die Feuchtigkeit des Gases beziehungsweise des Gasgemisches in dem Schüttelkolben einzustellen, ist vorzugsweise in die Leitung zwischen dem Einlass des Schüttelkolbens und der Gasmischbatterie eine Waschflasche eingeschaltet. Das von der Gasmischbatterie zugeführte Gas oder Gasgemisch wird mittels eines Tauchrohres gezwungen, durch eine Flüssigkeit in der Gaswaschflasche zu perlen, bevor es den Behälter wieder verlässt und über den Einlass in die Ausnehmung in der Begasungsklammer eingeleitet wird.

Wird bei der parallelen Kultivierung mehrerer Kulturen in einem Inkubator einer der Schüttelkolben aus dem Inkubator entnommen, hat dies auf die Begasung der Kulturen in den übrigen Schüttelkolben nahezu keinen Einfluss. Zwar ändert sich bei der Türöffnung des Inkubators die Temperatur geringfügig, jedoch hat diese geringe Temperaturänderung keinen Einfluss auf die Kulturen in den übrigen Schüttelkolben.

Es gibt auch Kultivierungen, die in einer Gasatmosphäre mit geringem Sauerstoffgehalt, beispielsweise 5%, durchgeführt werden müssen. Derartige Kultivierungen lassen sich mit der erfindungsgemäßen Begasungsvorrichtung vorteilhaft kultivieren, weil der beim Öffnen des Inkubators ansonsten zwangsläufig eintretende Anstieg der Sauerstoffkonzentration auf die Werte der Umgebungsluft vermieden werden kann. Zur Aufrechterhaltung der Gasatmosphäre in einem zu entnehmenden Schüttelkolben ist dessen Ein- und Auslass mit einem Absperrventil verschließbar. Die Aufrechterhaltung niedriger Sauerstoffkonzentrationen ist beispielsweise bei der Kultivierung von Stammzellen oder von mikroaerophilen Mikroorganismen erforderlich.

Indem die Feuchtigkeit der Gasatmosphäre in jedem individuell begasten Schüttelkolben mittels einer Waschflasche eingestellt wird, kann die Luftfeuchte im Innenraum des Inkubators geringer sein. Die geringere Feuchtigkeit in dem Innenraum des Inkubators erhöht dessen Lebensdauer.

Eine gute Abdichtung des Innenraums des Schüttelkolbens gegen die umgebende Atmosphäre sowie eine sichere Befestigung der Verschlusskappe an den Schüttelkolben wird in vorteilhafter Ausgestaltung der Erfindung dadurch erreicht, dass die Verschlusskappe als Schraubverschluss ausgebildet ist. Der Schraubverschluss ermöglicht zugleich eine einfache Befestigung eines kreisförmigen Sterilfilters, der entlang seines Umfangsrandes zwischen dem oberen Rand des Halses des Schüttelkolbens und der Innenseite der Decke der Verschlusskappe eingeklemmt wird. Diese Befestigung bewirkt zugleich die Abdichtung des Sterilfilters gegen die Innenseite der Decke der Verschlusskappe.

In einer anderen Ausgestaltung der Erfindung ist an der Innenseite der Verschlusskappe eine Halterung zur Aufnahme des Sterilfilters angeordnet. Vorzugsweise umgibt die Halterung den Gasdurchgang ringförmig und nimmt den Sterilfilter an seinem Umfangsrand bündig auf. Hierdurch wird der Sterilfilter nicht nur an der Innenseite der Decke der Verschlusskappe unterhalb des Gasdurchgangs befestigt, sondern zugleich gegen die Innenseite der Decke abgedichtet. Die sich von der Innenseite der Decke in die Verschlusskappe hinein erstreckende Halterung kann beim Spritzgießen der Verschlusskappe in einem Arbeitsgang hergestellt werden.

Eine ebenfalls im Wege des Spritzgießens einfach herstellbare und zudem gut handhabbare Begasungsklammer weist zwei diametral gegenüberliegende Klemmbacken auf.

Wenn die Krümmung der Klemmbacken an die Krümmung des üblicherweise kreiszylindrischen Mantels der Verschlusskappe angepasst ist, kann die Begasungsklammer in beliebiger Drehlage zur Längsmittelachse der Verschlusskappe aufgesetzt und gegenüber der Verschlusskappe verdreht werden. Dies erlaubt eine Ausrichtung des Ein- und Auslasses der Begasungsklammer unter Berücksichtigung der räumlichen Verhältnisse in dem Inkubator sowie der GasversorgungsLeitungen.
Um ein Abrutschen der Begasungsklammer von der Verschlusskappe zu verhindern und zugleich eine Vorspannung zwischen dem Steg der Begasungsklammer und der Decke der Verschlusskappe bei moderaten Klemmkräften auf den Mantel der Verschlusskappe zu erzeugen, weist jede Klemmbacke vorzugsweise eine Rastnase auf, die einen öffnungsseitigen Rand der Verschlusskappe hintergreift. Die Vorspannung ist erforderlich, um eine hinreichende Abdichtung der Ausnehmung der Begasungsklammer gegen die Außenseite der Verschlusskappe sicherzustellen.

Wenn die Ausnehmung den Gasdurchgang vollständig überdeckt, wird der Gasaustausch durch den Gasdurchgang in der Verschlusskappe verbessert. Ein ebener Rand, der die Ausnehmung vollständig umgibt, kann die Ausnehmung gegen die Außenseite wirksam abdichten.

Eine nochmals verbesserte Gasdichtigkeit wird dadurch erreicht, dass als Abdichtung der Ausnehmung gegen die Außenseite der Decke der Verschlusskappe ein die Ausnehmung umgebender O-Ring zwischen der Decke und dem Steg der Begasungsklammer angeordnet ist. Der O-Ring ist vorzugsweise in eine umlaufende Nut an der Innenseite des Stegs eingelassen. Die Nut wird vorzugsweise in einen ebenen Rand eingelassen, der die Ausnehmung vollständig umgibt.

In vorteilhafter Ausgestaltung der Erfindung sind die Ausnehmung und der Gasdurchgang fluchtend zu der Längsmittelachse der Verschlusskappe angeordnet. Sofern sowohl die Ausnehmung als auch der Gasdurchgang im Querschnitt kreisförmig sind, wird durch die Ausrichtung an der Längsmittelachse ein rotationssymmetrischer Gasraum geschaffen, in dem sich ein gleichmäßiges Mikroklima oberhalb des Sterilfilters ausbildet.

Zur weiteren Vergleichmäßigung des Mikroklimas trägt eine Anordnung des Ein- und Auslasses diametral zur Längsmittelachse der Verschlusskappe bei. Der Ein- und Auslass sind insbesondere als hohlzylindrische Durchgänge ausgebildet, die in der Ausnehmung münden. Die Zylinderachsen der Durchgänge verlaufen parallel zur Längsmittelachse in einem möglichst großen Abstand.

Nachfolgend wir die Erfindung anhand der Figuren näher erläutert.

Es zeigen
- **Fig. 1A**: eine Aufsicht auf ein erstes Ausführungsbeispiel einer erfindungsgemäßen Begasungsvorrichtung,
- **Fig. 1B**: einen Schnitt entlang der Linie A-A nach Fig. 1a,
- **Fig. 1C**: einen Schnitt entlang der Linie B-B nach Fig. 1a,
- **Fig. 1D**: einen Schnitt entlang der Linie C-C nach Fig. 1a,
- **Fig. 2A**: eine Aufsicht auf ein zweites Ausführungsbeispiel der erfindungsgemäßen Begasungsvorrichtung,
- **Fig. 2B**: einen Schnitt entlang der Linie B-B nach Fig. 2a,
- **Fig. 2C**: einen Schnitt entlang der Linie C-C nach Fig. 2a sowie
- **Fig. 3**: eine schematische Darstellung eines Schüttel-Inkubators mit mehreren Schüttelkolben, die mit erfindungsgemäßen Begasungsvorrichtungen ausgerüstet sind.

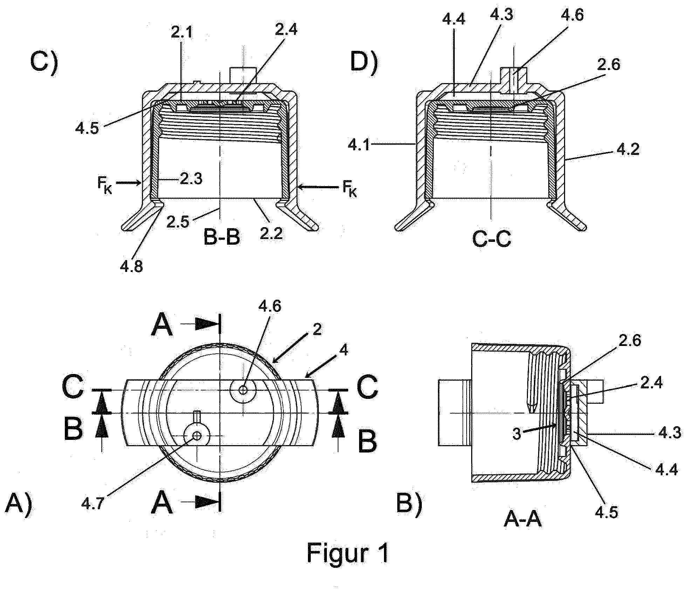

Die Begasungsvorrichtung für einen Schüttelkolben (1) umfasst eine hohlzylindrische, als Schraubverschluss ausgebildete Verschlusskappe (2) mit einer Decke (2.1), einer der Decke (2.1) gegenüberliegenden unteren Öffnung (2.2) sowie einem sich zwischen der Decke (2.1) und der unteren Öffnung (2.2) erstreckenden Mantel (2.3). In der Decke (2.1) ist ein in den Figuren 1B und 1C erkennbarer Gasdurchgang (2.4) konzentrisch zur Längsmittelachse (2.5) der Verschlusskappe (2) angeordnet. Der Gasdurchgang (2.4) weist radiale und ringförmig verlaufende Aussteifungen auf, die einteilig mit der Verschlusskappe (2) als Spritzgussteil hergestellt werden.

An der Innenseite der Verschlusskappe (2) ist eine ringförmig den Gasdurchgang (2.4) umgebende Halterung (2.6) an die Innenseite der Verschlusskappe (2) angespritzt, die einen Sterilfilter (3) bündig aufnimmt. Die angespritzte Halterung (2.6) dichtet den Sterilfilter zugleich gegen die Innenseite der Decke (2.1) der Verschlusskappe (2) ab.

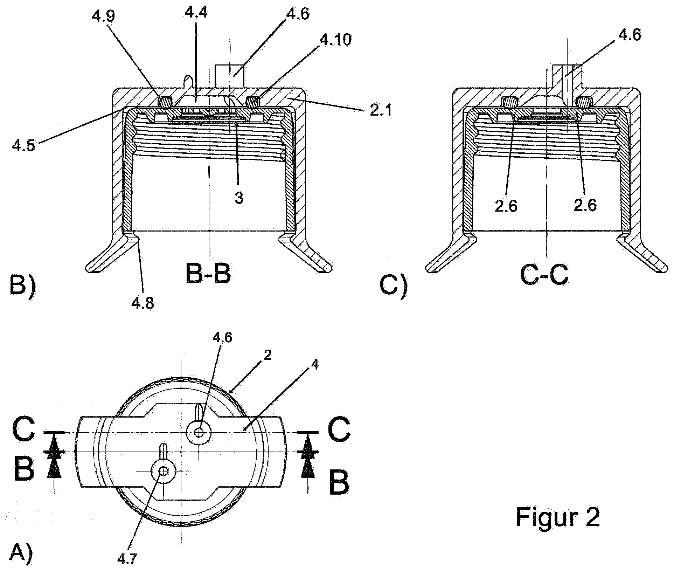

Mit der Verschlusskappe (2) ist eine Begasungsklammer (4) lösbar verbunden. Figuren 1 und 2 zeigen jeweils eine Verschlusskappe (2), die korrekt mit der Begasungsklammer (4) verbunden ist. Die Begasungsklammer (2) weist zwei diametral gegenüberliegende, elastische Klemmbacken (4.1, 4.2) und einen die beiden Klemmbacken miteinander verbindenden Steg (4.3) auf, wobei die Klemmbacken (4.1, 4.2) auf den Mantel (2.3) der Verschlusskappe (2) eine Klemmkraft (F_{K}) ausüben.

Der Steg (4.3) weist eine der Außenseite der Decke (2.1) der Verschlusskappe (2) zugewandte Innenseite auf. An der Innenseite des Stegs (4.3) befindet sich eine Ausnehmung (4.4) oberhalb des Gasdurchgangs (2.4). Wie insbesondere aus Figur 1C in Verbindung mit Figur 1B erkennbar, überdeckt die Ausnehmung (4.4) den Gasdurchgang (2.4) vollständig. Ein ebener Rand (4.5) umgibt die Ausnehmung (4.4) vollständig, wobei der ebene Rand (4.5) in Längsrichtung des Steges (4.3) geradlinig, wie aus Figur 1B erkennbar, und im Übergangsbereich zu den Klemmbacken (4.1, 4.2) gekrümmt verläuft. Der ebene Rand (4.5) liegt bündig an der ebenen Außenseite der Decke (2.1) der Verschlusskappe (2) an und bewirkt dadurch eine Abdichtung der Ausnehmung (4.4) gegen die Verschlusskappe (2).

In die Ausnehmung (4.4) der Begasungsklammer (4) münden zwei zylindrische Durchgänge, wobei einer der beiden Durchgänge einen Einlass (4.6) zur Zufuhr von Gas in die Ausnehmung (4.4) und der andere Durchgang ein Auslass für das Austreten von Gas aus der Ausnehmung (4.4) bildet.

Wie insbesondere aus der Aufsicht nach Figur 1A erkennbar, ist die Krümmung der Klemmbacken (4.1, 4.2) an die Krümmung des im Querschnitt kreiszylindrischen Mantels (2.3) der Verschlusskappe (2) angepasst. Beide Klemmbacken (4.1, 4.2) sind an ihren unteren, freien Enden nach außen abgewinkelt. Im Bereich der Abwinkelung weist jede Klemmbacke (4.1, 4.2) eine Rastnase (4.8) auf, die den Rand an der unteren Öffnung (2.2) der Verschlusskappe (2) hintergreift.

Die Begasungsvorrichtung nach Figur 2 entspricht im Aufbau der Begasungsvorrichtung nach Figur 1. Übereinstimmende Bauteile sind daher mit übereinstimmenden Bezugszeichen versehen. Unterschiede ergeben sich jedoch hinsichtlich der Form der Ausnehmung (4.4) sowie deren Abdichtung gegen die Außenseite der Decke (2.1) der Verschlusskappe (2). Die Ausnehmung (4.4) weist einen kreisförmigen Querschnitt auf, der von einem ebenen, ringförmig die Ausnehmung (4.4) umgebenden Rand (4.5) vollständig umgeben wird. In den ebenen Rand ist eine ringförmig umlaufende Nut (4.9) in den Steg (4.3) eingelassen. Die Nut (4.9) nimmt einen O-Ring (4.10) auf, der die Ausnehmung (4.4) gegen die Außenseite der Decke (2.1) abdichtet.

Um den Abstand zwischen den den Einlass (4.6) und den Auslass (4.7) bildenden Durchgängen zu der Längsmittelachse (2.5) der Verschlusskappe (2) so groß wie möglich zu wählen, münden die Durchgänge in den sich konisch vom Grund der Ausnehmung erweiternden Seitenwänden der Ausnehmung (4.4).

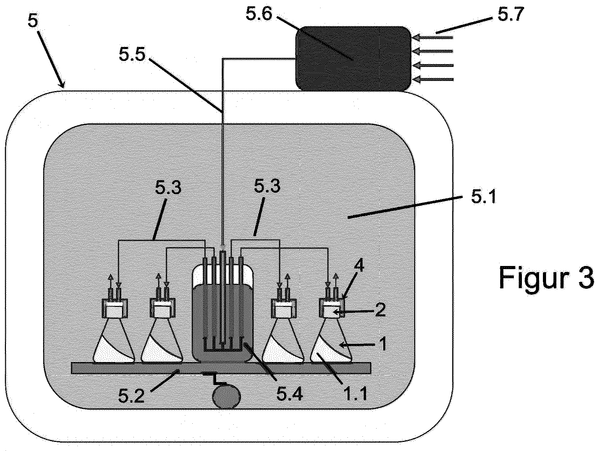

Figur 3 zeigt einen Schüttel-Inkubator (5) mit einem Innenraum (5.1), in dem auf einem Schüttler (5.2) vier Schüttelkolben (1) fixiert sind, die mit einer erfindungsgemäßen Begasungsvorrichtung umfassend eine Verschlusskappe (2) sowie eine Begasungsklammer (4) ausgestattet sind. Die Einlässe (4.6) der vier Schüttelkolben (1) sind über lediglich schematisch dargestellte Schlauchleitungen (5.3) mit einem Gasverteiler und -wäscher (5.4) gasleitend verbunden. Der Gasverteiler und -wäscher (5.4) ist über eine Gaszuführleitung (5.5) mit einer Gasmischbatterie (5.6) verbunden, die das Gas aus mehreren Gasquellen (5.7) für unterschiedliche Gase beziehungsweise Gasgemische, wie beispielsweise Luft, Stickstoff, Sauerstoff und Kohlendioxid entsprechend der für die Begasung benötigten Gasatmosphäre vermischt. Derartige Gasmischmodule (5.6) sind an sich bekannt und werden beispielsweise von der Adolf Kühner AG, Dinkelbergstrasse 1, CH-4127 Birsfelden (Basel) unter der Bezeichnung FlowCon 2/3/4 angeboten (vgl. Kuhner shaker, FlowCon 2/3/4 Stand-alone gas mixing device, https://www.kuhner.com/de/produkte/anwendungstechnologien/gas -mixing/flowcon-234.html, heruntergeladen am 30.11.2018).

Die Gasmischstation (5.6) sowie die Gasquellen (5.7) sind außerhalb des Schüttel-Inkubators (5) angeordnet, lediglich die Gaszuführleitung (5.5) durchsetzt die Wand des Schüttel-Inkubators (5). Das Gas beziehungsweise das Gasgemisch gelangt über die Gaszuführleitung (5.5) zu dem Gasverteiler und -wäscher (5.4). Dieser verteilt das Gas unabhängig von der Gasatmosphäre in dem Innenraum (5.1) des Schüttel-Inkubators (5) über die Leitungen (5.3) zu den Begasungsvorrichtungen der einzelnen Schüttelkolben (1).

In dem Gasraum oberhalb des Sterilfilters (3) der Begasungsvorrichtung jedes Schüttelkolbens (1) bildet sich ein Mikroklima aus, was die zugeführte Gaszusammensetzung und die durch den Gasverteiler und -wäscher (5.4) eingestellte Feuchtigkeit aufweist. Unter der Wirkung dieses Mikroklimas erfolgt die Kultivierung der Kulturmedien (1.1) in den Schüttelkolben (1). Wird nun ein Schüttelkolben (1) dem Schüttel-Inkubator (5) entnommen, hat diese Entnahme praktisch keinen Einfluss auf die parallelen Kultivierungen. Die geringfügige Abnahme der Temperatur in dem Innenraum (5.1) durch das Öffnen der Inkubator-Tür wirkt sich auf die Kultivierung der Kulturmedien (1.1) in den verbleibenden Schüttelkolben (1) praktisch nicht aus. Durch die besonders einfach handhabbare und von den Verschlusskappen (2) lösbare Begasungsklammer (4) kann auf einfache Art und Weise jeder Schüttelkolben mit einer erfindungsgemäßen Begasungsvorrichtung versehen werden.

Sofern nach dem Trennen der erfindungsgemäßen Begasungsvorrichtung von der Leitung (5.3) für die Kultivierung die Gasatmosphäre in dem Schüttelkolben (1) aufrechterhalten werden soll, kann in einer nicht dargestellten Ausführungsform der Erfindung sowohl der Einals auch der Auslass (4.6, 4.7) jeweils mit einem Absperrventil versehen sein.

Hierzu können manuell betätigbare Absperrventile oder Quetschventile an Schlauchleitungen vorgesehen sein, die mit dem Ein- beziehungsweise Auslass in Verbindung stehen.

**Bezugszeichenliste**

| **Nr.** | **Bezeichnung** |
|---|---|
| 1 | Schüttelkolben |
| 1.1 | Kulturmedien |
| 2 | Verschlusskappe |
| 2.1 | Decke |
| 2.2 | Untere Öffnung |
| 2.3 | Mantel |
| 2.4 | Gasdurchgang |
| 2.5 | Längsmittelachse |
| 2.6 | Halterung |
| 3 | Sterilfilter |
| 4 | Begasungsklammer |
| 4.1 | Klemmbacke |
| 4.2 | Klemmbacke |
| 4.3 | Steg |
| 4.4 | Ausnehmung |
| 4.5 | Ebener Rand |
| 4.6 | Einlass |
| 4.7 | Auslass |
| 4.8 | Rastnase |
| 4.9 | Nut |
| 4.10 | O-Ring |
| 4.11 | Seitenwand |
| 5 | Schüttel-Inkubator |
| 5.1 | Innenraum |
| 5.2 | Schüttler |
| 5.3 | Leitungen |
| 5.4 | Gasverteiler und -wäscher |
| 5.5 | Gaszuführleitung |
| 5.6 | Gasmischbatterie |
| 5.7 | Gasquellen |

## Patentansprüche

1. Begasungsvorrichtung für einen Schüttelkolben (1) umfassend
- eine hohlzylindrische Verschlusskappe (2) mit einer Decke (2.1), einer unteren Öffnung (2.2) sowie einem sich zwischen Decke (2.1) und der unterer Öffnung (2.2) erstreckenden Mantel (2.3),
- einen Gasdurchgang (2.4) in der Decke (2.1),
- einen an einer Innenseite der Decke (2.1) unterhalb des Gasdurchgangs (2.4) angeordneten Sterilfilter (3),
- eine Abdichtung des Sterilfilters (3) gegen die Innenseite der Decke (2.1),
- eine lösbar mit der Verschlusskappe (2) verbundene Begasungsklammer (4) mit mindestens zwei elastischen Klemmbacken (4.1, 4.2) und einem die Klemmbacken miteinander verbindenden Steg (4.3), wobei die Klemmbacken (4.1, 4.2) auf den Mantel (2.3) eine Klemmkraft (F_{K}) ausüben,
- eine Ausnehmung (4.4) an einer Innenseite des Stegs (4.3), wobei die Ausnehmung (4.4) oberhalb des Gasdurchgangs (2.4) angeordnet ist,
- eine Abdichtung der Ausnehmung (4.4) gegen die Außenseite der Decke (2.1) und
- einen Einlass (4.6) zur Zufuhr von Gas in die Ausnehmung (4.4) und einen Auslass (4.7) für das Austreten von Gas aus der Ausnehmung (4.4).

2. Begasungsvorrichtung nach Anspruch 1, **dadurch gekennzeichnet, dass** die Verschlusskappe (2) als Schraubverschluss ausgebildet ist.

3. Begasungsvorrichtung nach Anspruch 1 oder 2, **dadurch gekennzeichnet, dass** an der Innenseite der Verschlusskappe (2) eine Halterung (2.6) zur Aufnahme eines Sterilfilters (3) angeordnet ist.

4. Begasungsvorrichtung nach Anspruch 3, **dadurch gekennzeichnet, dass** die Halterung (2.6) den Gasdurchgang (2.4) umgibt und den Sterilfilter (3) an seinem Umfangsrand bündig aufnimmt.

5. Begasungsvorrichtung nach einem der Ansprüche 1 bis 4, **dadurch gekennzeichnet, dass** die Begasungsklammer (4) zwei diametral gegenüberliegende Klemmbacken (4.1, 4.2) aufweist.

6. Begasungsvorrichtung nach einem der Ansprüche 1 bis 5, **dadurch gekennzeichnet, dass** die Krümmung der Klemmbacken (4.1, 4.2) an die Krümmung des Mantels (2.3) der Verschlusskappe (2) angepasst ist.

7. Begasungsvorrichtung nach einem der Ansprüche 1 bis 6, **dadurch gekennzeichnet, dass** jede Klemmbacke (4.1, 4.2) eine Verrastung (4.8) aufweist, die einen öffnungsseitigen Rand der Verschlusskappe (2) hintergreift.

8. Begasungsvorrichtung nach einem der Ansprüche 1 bis 7, **dadurch gekennzeichnet, dass** die Ausnehmung (4.4) den Gasdurchgang (2.4) vollständig überlappt.

9. Begasungsvorrichtung nach einem der Ansprüche 1 bis 8, **dadurch gekennzeichnet, dass** ein ebener Rand die Ausnehmung (4.4) vollständig umgibt.

10. Begasungsvorrichtung nach einem der Ansprüche 1 bis 9, **dadurch gekennzeichnet, dass** als Abdichtung der Ausnehmung (4.4) gegen die Außenseite der Decke (2.1) ein die Ausnehmung umgebender O-Ring (4.10) zwischen der Decke (2.1) und dem Steg (4.3) angeordnet ist.

11. Begasungsvorrichtung nach einem der Ansprüche 1 bis 10, **dadurch gekennzeichnet, dass** die Ausnehmung (4.4) und der Gasdurchgang (2.4) fluchtend zu einer Längsmittelachse (2.5) der Verschlusskappe (2) angeordnet sind.

12. Begasungsvorrichtung nach einem der Ansprüche 1 bis 11, **dadurch gekennzeichnet, dass** Einlass (4.6) und Auslass (4.7) diametral zur Längsmittelachse (2.5) der Verschlusskappe (2) angeordnet sind.

13. Begasungsvorrichtung nach einem der Ansprüche 1 bis 12, **dadurch gekennzeichnet, dass** der Einlass (4.6) über eine Leitung (5.3, 5.5) mit einer Gasmischbatterie (5.6) verbunden ist.

14. Begasungsvorrichtung nach Anspruch 13, **dadurch gekennzeichnet, dass** in die Leitung (5.3, 5.5) eine Waschflasche (5.4) eingeschaltet ist.

15. Begasungsvorrichtung nach einem der Ansprüche 1 bis 14, **dadurch gekennzeichnet, dass** der Einlass (4.6) und/oder der Auslass (4.7) mittels eines Absperrventils verschließbar sind.

16. Schüttel-Inkubator (5) mit einem Innenraum (5.1), in dem mehrere Schüttelkolben (1) mit einer Begasungsvorrichtung nach einem oder mehreren der Ansprüche 1 bis 15 angeordnet sind.

## Claims

1. Gassing device for a shake flask (1) comprising
- a hollow-cylindrical closure cap (2) having a cover (2.1), a lower opening (2.2) and a casing (2.3) extending between cover (2.1) and the lower opening (2.2),
- a gas passage (2.4) in the cover (2.1),
- a sterile filter (3) arranged on an inner side of the cover (2.1) below the gas passage (2.4),
- a seal of the sterile filter (3) against the inner side of the cover (2.1),
- a gassing clamp (4) connected detachably to the closure cap (2) having at least two elastic clamping jaws (4.1, 4.2) and a crosspiece (4.3) connecting the clamping jaws to one another, wherein the clamping jaws (4.1, 4.2) exert a clamping force (F_{K}) on the casing (2.3),
- a recess (4.4) on an inner side of the crosspiece (4.3), wherein the recess (4.4) is arranged above the gas passage (2.4),
- a seal of the recess (4.4) against the outer side of the cover (2.1) and
- an inlet (4.6) for supplying gas into the recess (4.4) and an outlet (4.7) for the escape of gas from the recess (4.4).

2. Gassing device according to Claim 1, **characterized in that** the closure cap (2) is configured as a screw closure.

3. Gassing device according to Claim 1 or 2, **characterized in that** a holder (2.6) for receiving a sterile filter (3) is arranged on the inner side of the closure cap (2).

4. Gassing device according to Claim 3, **characterized in that** the holder (2.6) surrounds the gas passage (2.4) and receives the sterile filter (3) flush at its circumferential edge.

5. Gassing device according to one of Claims 1 to 4, **characterized in that** the gassing clamp (4) comprises two diametrically opposite clamping jaws (4.1, 4.2).

6. Gassing device according to one of Claims 1 to 5, **characterized in that** the curvature of the clamping jaws (4.1, 4.2) is adapted to the curvature of the casing (2.3) of the closure cap (2).

7. Gassing device according to one of Claims 1 to 6, **characterized in that** each clamping jaw (4.1, 4.2) has a catch mechanism (4.8) which engages behind an opening-side edge of the closure cap (2).

8. Gassing device according to one of Claims 1 to 7, **characterized in that** the recess (4.4) completely overlaps the gas passage (2.4).

9. Gassing device according to one of Claims 1 to 8, **characterized in that** a flat edge completely surrounds the recess (4.4).

10. Gassing device according to one of Claims 1 to 9, **characterized in that** an O-ring (4.10) surrounding the recess is arranged between the cover (2.1) and the crosspiece (4.3) as a seal of the recess (4.4) against the outer side of the cover (2.1).

11. Gassing device according to one of Claims 1 to 10, **characterized in that** the recess (4.4) and the gas passage (2.4) are arranged in alignment with a longitudinal central axis (2.5) of the closure cap (2) .

12. Gassing device according to one of Claims 1 to 11, **characterized in that** inlet (4.6) and outlet (4.7) are arranged diametrically opposite the longitudinal central axis (2.5) of the closure cap (2).

13. Gassing device according to one of Claims 1 to 12, **characterized in that** the inlet (4.6) is connected to a gas mixing battery (5.6) via a line (5.3, 5.5).

14. Gassing device according to Claim 13, **characterized in that** a washing bottle (5.4) is connected into the line (5.3, 5.5).

15. Gassing device according to one of Claims 1 to 14, **characterized in that** the inlet (4.6) and/or the outlet (4.7) can be closed by means of a check valve.

16. Shake incubator (5) having an interior (5.1) in which a plurality of shake flasks (1) having a gassing device according to one or more of Claims 1 to 15 are arranged.

## Revendications

1. Dispositif fumigateur pour une fiole agitée (1), comprenant
- un capuchon de fermeture (2) cylindrique creux, pourvu d'un recouvrement (2.1), d'une ouverture (2.2) inférieure, ainsi que d'une enveloppe (2.3) s'étendant entre le recouvrement (2.1) et l'ouverture (2.2) inférieure,
- un passage de gaz (2.4) dans le recouvrement (2.1),
- un filtre stérile (3), placé sur une face intérieure du recouvrement (2.1), en-dessous du passage de gaz (2.4),
- une garniture d'étanchéité pour le filtre stérile (3) à l'encontre de la face intérieure du recouvrement (2.1),
- une pince de fumigation (4) reliée de manière amovible avec le capuchon de fermeture (2), dotée d'au moins deux mâchoires de serrage (4.1, 4.2) élastiques, d'un listel (4.3) reliant entre elles les mâchoires de serrage, les mâchoires de serrage (4.1, 4.2) exerçant une force de serrage (F_{K}) sur l'enveloppe (2.3),
- un évidement (4.4) sur une face intérieure du listel (4.3), l'évidement (4.4) étant placé au-dessus du passage de gaz (2.4),
- une garniture d'étanchéité pour l'évidement (4.4) à l'encontre de la face extérieure du recouvrement (2.1) et
- une entrée (4.6) pour l'alimentation de gaz dans l'évidement (4.4) et une sortie (4.7) pour l'échappement de gaz hors de l'évidement (4.4).

2. Dispositif fumigateur selon la revendication 1, **caractérisé en ce que** le capuchon de fermeture (2) est conçu sous la forme d'une fermeture filetée.

3. Dispositif fumigateur selon la revendication 1 ou 2, **caractérisé en ce que** sur la face intérieure du capuchon de fermeture (2) est placée une fixation (2.6), destinée à réceptionner un filtre stérile (3).

4. Dispositif fumigateur selon la revendication 3, **caractérisé en ce que** la fixation (2.6) entoure le passage de gaz (2.4) et réceptionne à fleur le filtre stérile (3) sur son bord périphérique.

5. Dispositif fumigateur selon l'une quelconque des revendications 1 à 4, **caractérisé en ce que** la pince de fumigation (4) comporte deux mâchoires de serrage (4.1, 4.2) diamétralement opposées.

6. Dispositif fumigateur selon l'une quelconque des revendications 1 à 5, **caractérisé en ce que** la courbure des mâchoires de serrage (4.1, 4.2) est adaptée à la courbure de l'enveloppe (2.3) du capuchon de fermeture (2).

7. Dispositif fumigateur selon l'une quelconque des revendications 1 à 6, **caractérisé en ce que** chaque mâchoire de serrage (4.1, 4.2) comporte un encliquetage (4.8) qui s'accroche par l'arrière dans un bord du côté de l'ouverture du capuchon de fermeture (2).

8. Dispositif fumigateur selon l'une quelconque des revendications 1 à 7, **caractérisé en ce que** l'évidement (4.4) chevauche totalement le passage de gaz (2.4).

9. Dispositif fumigateur selon l'une quelconque des revendications 1 à 8, **caractérisé en ce qu'**un bord plan entoure totalement l'évidement (4.4).

10. Dispositif fumigateur selon l'une quelconque des revendications 1 à 9, **caractérisé en ce qu'**en tant que garniture d'étanchéité de l'évidement (4.4) à l'encontre de la face extérieure du recouvrement (2.1), une bague torique (4.10) entourant l'évidement est placée entre le recouvrement (2.1) et le listel (4.3) .

11. Dispositif fumigateur selon l'une quelconque des revendications 1 à 10, **caractérisé en ce que** l'évidement (4.4) et le passage de gaz (2.4) sont placés en alignement sur un axe longitudinal médian (2.5) du capuchon de fermeture (2).

12. Dispositif fumigateur selon l'une quelconque des revendications 1 à 11, **caractérisé en ce que** l'entrée (4.6) et la sortie (4.7) sont placées diamétralement à l'axe longitudinal médian (2.5) du capuchon de fermeture (2).

13. Dispositif fumigateur selon l'une quelconque des revendications 1 à 12, **caractérisé en ce que** l'entrée (4.6) est reliée par l'intermédiaire d'un conduit (5.3, 5.5) avec un robinet mélangeur de gaz (5.6).

14. Dispositif fumigateur selon la revendication 13, **caractérisé en ce que** dans le conduit (5.3, 5.5) est connecté un flacon de lavage (5.4).

15. Dispositif fumigateur selon l'une quelconque des revendications 1 à 14, **caractérisé en ce que** l'entrée (4.6) et/ou la sortie (4.7) sont susceptibles d'être fermées au moyen d'une vanne d'arrêt.

16. Incubateur agitateur (5) pourvu d'un espace intérieur (5.1), dans lequel sont placés plusieurs fioles agitées (1) pourvues d'un dispositif fumigateur selon l'une ou plusieurs des revendications 1 à 15.
